# EUROPÄISCHE PATENTSCHRIFT

(11) **EP 0 496 113 B1**
(45) Veröffentlichungstag und Bekanntmachung des Hinweises auf die Patenterteilung: **22.11.1995**
(21) Anmeldenummer: 91122251.1
(22) Anmeldetag: 27.12.1991
(51) Int. Cl.: C09B 1/503, C07C 225/36

(54) **Verfahren zur Herstellung von 1-Amino-2-chlor-4-hydroxi-anthrachinon**
Process for the preparation of 1-amino-2-chloro-4-hydroxy-anthraquinone
Procédé de préparation de la 1-amino-2-chloro-4-hydroxyanthraquinone

(30) Priorität: 23.01.1991 DE 4101875
(43) Veröffentlichungstag der Anmeldung: 29.07.1992
(73) Patentinhaber: BASF Aktiengesellschaft, 67063 Ludwigshafen (DE)
(72) Erfinder: Epple, Gerhard, Dr., W-6719 Weisenheim (DE)

(56) Entgegenhaltungen:
- EP-A- 0 361 253
- DE-A- 2 428 337
- GB-A- 507 065

## Beschreibung

Die Erfindung betrifft ein neues Verfahren zur Herstellung von 1-Amino-2-chlor-4-hydroxi-anthrachinon I durch Chlorierung von 1-Amino-4-hydroxi-2,3-dihydro-anthrachinon II. 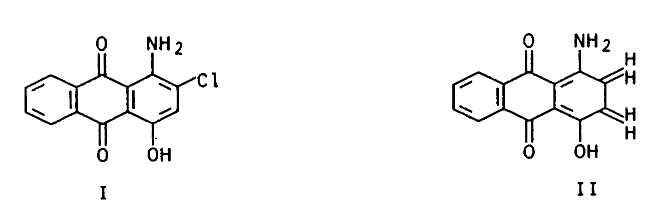
Aus der DE-OS 38 32 740 ist bekannt, daß man 1-Amino-2-chlor-4-hydroxianthrachinon durch Chlorierung von 1-Amino-4-hydroxi-anthrachinon III in 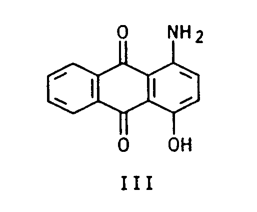
90 bis 100 %iger wäßriger Schwefelsäure oder in bis zu 2 %igem Oleum herstellen kann.

Ausgehend von 1,4-Dihydroxi-anthrachinon IV (Chinizarin) erfordert die Herstellung von genügend reinem III jedoch mindestens zwei Synthesestufen, nämlich die Umsetzung zu 1,4-Diamino-2,3-dihydro-anthrachinon V (Leukamin) in Ammoniak und die anschließende Umsetzung zu III in Schwefelsäure mit Braunstein. Wegen der Verwendung von Braunstein entstehen Probleme bei der Regenerierung der Schwefelsäure. 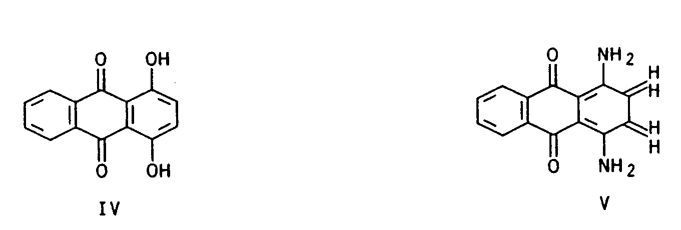
Die Herstellung von II ist aus der französischen Patentschrift 602 882 oder der britischen Patentschrift 507 065 bekannt.

In der Regel führt man das Verfahren gemäß der Erfindung so durch, daß man das Ausgangsprodukt II in der 4- bis 20fachen, vorzugsweise 6- bis 10fachen, Menge an 92 bis 100 %iger, vorzugsweise 96 bis 99 %iger, Schwefelsäure unter Rühren löst und bei 20 bis 80°C, vorzugsweise 40 bis 70°C, Chlor einleitet, wobei mindestens 2 Mol Chlor erforderlich sind.

Der Reaktionsverlauf wird zweckmäßigerweise dünnschichtchromatographisch überwacht. Oxidation von II zu III und Chlorierung von III verlaufen nebeneinander. Die Reaktion wird unterbrochen, wenn II vollständig und mehr als 97 % von III umgesetzt sind, indem man das Reaktionsgemisch entweder in Wasser gießt oder langsam mit Wasser versetzt und dabei das Reaktionsprodukt ausfällt.

1-Amino-2-chlor-4-hydroxi-anthrachinon ist ein wichtiges Zwischenprodukt bei der Herstellung von roten Anthrachinonfarbstoffen, z.B. von Disperse Red 60, C.I 60756.

Die folgenden Beispiele sollen das Verfahren gemäß der Erfindung weiter erläutern.

### Beispiel 1

In 3000 g 97,5 %ige Schwefelsäure werden 500 g 95 %iges 1-Amino-4-hydroxi-2,3-dihydro-anthrachinon (II) unter Rühren eingetragen. Nach dem Erwärmen auf 60°C werden innerhalb von 24 Stunden 308 g Chlor eingeleitet. Anschließend wird 2 Stunden Stickstoff durch das Reaktionsgemisch geleitet, um gelösten Chlorwasserstoff auszutreiben.

Danach werden bei 60°C in 3 Stunden 1220 g Wasser zugetropft, wobei das Reaktionsprodukt ausfällt. Es wird abgesaugt, mit 1500 g 60 %iger Schwefelsäure und anschließend mit Wasser neutral gewaschen und getrocknet.
- Ausbeute:: 509,8 g 92,3 %iges Produkt, das sind 87,3 % d.Th.

### Beispiel 2

In 2400 g 97,4 %iger Schwefelsäure werden 440 g 90,9 %iges 1-Amino-4-hydroxi-2,3-dihydro-anthrachinon (II) gelöst. Bei 60°C wird so lange Chlor eingeleitet, bis dünnschichtchromatographisch nur noch Spuren an 1-Amino-4-hydroxi-anthrachinon III nachweisbar sind. Nach 2stündigem Durchleiten von Stickstoff werden dann bei 60 bis 90°C 960 g Wasser innerhalb von 2 Stunden zugetropft. Das Reaktionsgemisch wird abgekühlt und das ausgefallene Reaktionsprodukt abgesaugt. Es wird mit 2000 g 60 %iger Schwefelsäure, anschließend mit Wasser nachgewaschen und getrocknet.
- Ausbeute:: 456,6 g 90,9 %iges Produkt, das sind 91,4 % d.Th.

### Beispiel 3

In 506 g 97,5 %iger Schwefelsäure werden 80 g 95,6 %iges 1-Amino-4-hydroxi-2,3-dihydro-anthrachinon gelöst und wie in Beispiel 2 angegeben chloriert. Das Reaktionsprodukt wird durch Zutropfen von 210 g Wasser ausgefällt und wie in Beispiel 2 aufgearbeitet.
- Ausbeute:: 85,6 g 96,5 %iges Produkt, das sind 91 % d.Th.

### Beispiel 4

In 6000 g 97,5 %ige Schwefelsäure werden 1072 g 93,3 %iges 1-Amino-4-hydroxi-2,3-dihydroanthrachinon eingetragen und auf 60°C erwärmt. Bei 60-65°C werden innerhalb von 18 Stunden 700 g Chlor eingeleitet. Anschließend wird Stickstoff durch das Reaktionsgemisch geleitet, um gelösten Chlorwasserstoff auszutreiben.

Bei 90-95°C läßt man dann 2400 g Wasser zulaufen, wodurch das Reaktionsprodukt ausfällt, das nach dem Abkühlen auf 20-25°C abfiltriert und mit Schwefelsäure und Wasser gewaschen wird.

Nach dem Trocknen erhält man 1033 g 92 %iges 1-Amino-2-chlor-4-hydroxi-anthrachinon.

## Patentansprüche

1. Verfahren zur Herstellung von 1-Amino-2-chlor-4-hydroxi-anthrachinon (I), dadurch gekennzeichnet, daß man 1-Amino-4-hydroxi-2,3-dihydro-anthrachinon (II) in konzentrierter Schwefelsäure mit mindestens 2 Mol Chlor chloriert.

2. Verfahren gemäß Anspruch 1, dadurch gekennzeichnet, daß man die 4- bis 20fache Menge an 92 bis 100 %iger Schwefelsäure, bezogen auf das Dihydroanthrachinon, verwendet.

3. Verfahren gemäß Anspruch 1, dadurch gekennzeichnet, daß man die Chlorierung bei Temperaturen von 40 bis 70°C durchführt.

## Claims

1. A process for preparing 1-amino-2-chloro-4-hydroxyanthraquinone (I), which comprises chlorinating 1-amino-4-hydroxy-2,3-dihydroanthraquinone (II) with at least 2 mol of chlorine in concentrated sulfuric acid.

2. A process as claimed in claim 1, wherein from 4 to 20 times the amount of 92-100 % sulfuric acid is used, based on the dihydroanthraquinone.

3. A process as claimed in claim 1, wherein the chlorination is carried out at from 40 to 70°C.

## Revendications

1. Procédé de préparation de 1-amino-2-chloro-4-hydroxyanthraquinone (I), caractérisé en ce que l'on chlore de la 1-amino-4-hydroxy-2,3-dihydroanthraquinone (II) dans de l'acide sulfurique concentré, avec au moins 2 moles de chlore.

2. Procédé selon la revendication 1, caractérisé en ce que l'on utilise de l'acide sulfurique à 92-100% dans une proportion de 4 à 20 fois par rapport à la dihydroanthraquinone.

3. Procédé selon la revendication 1, caractérisé en ce que l'on effectue la chloration à des températures de 40 à 70°C.
